Europäisches Patentamt

European Patent Office

Office européen des brevets

(11) Veröffentlichungsnummer: **0 343 542 B1**

## (12) EUROPÄISCHE PATENTSCHRIFT

(45) Veröffentlichungstag der Patentschrift: **23.12.92**

(21) Anmeldenummer: **89109110.0**

(22) Anmeldetag: **20.05.89**

(51) Int. Cl.5: **A61M 16/00**

(54) **Gerät zur Unterstützung der spontanen Atmung eines Patienten.**

(30) Priorität: **27.05.88 DE 3817985**

(43) Veröffentlichungstag der Anmeldung:
**29.11.89 Patentblatt 89/48**

(45) Bekanntmachung des Hinweises auf die
Patenterteilung:
**23.12.92 Patentblatt 92/52**

(84) Benannte Vertragsstaaten:
**AT BE CH DE ES FR GB GR IT LI LU NL SE**

(56) Entgegenhaltungen:
**FR-A- 2 565 100**
**GB-A- 2 054 387**

(73) Patentinhaber: **HESTIA PHARMA GMBH**
**Neckarauerstrasse 152-162**
**W-6800 Mannheim 1(DE)**

(72) Erfinder: **Breitenfelder, Wilhelm**
**Gebrüder-Lang-Strasse 18**
**W-6360 Friedberg(DE)**
Erfinder: **Trenkner, Fritz**
**Lübecker Strasse 4**
**W-6336 Eschborn/Taunus(DE)**

(74) Vertreter: **Pfeifer, Hans-Peter, Dr.rer.nat.**
**Patentanwalt Nowackanlage 15**
**W-7500 Karlsruhe 1(DE)**

## Beschreibung

Die Erfindung betrifft ein Gerät zur Unterstützung der spontanen Atmung eines Patienten mit einer Atemgasmischeinheit zur Mischung von Luft und Sauerstoff in einem gewünschten Verhältnis zur Versorgung des Patienten mit Atemgas einer einstellbaren Sauerstoffkonzentration, einem Auslaß, an dem ein Druckbegrenzer angeordnet ist, einem Gaskanal, der die Atemgasmischeinheit mit dem Auslaß verbindet und einer Patientenleitung, die an einer zwischen der Atemgasmischeinheit und dem Auslaß angeordneten Abzweigung von dem Gaskanal abzweigt.

Derartige Geräte sind allgemein bekannt und am Markt erhältlich.

Patienten, die spontan atmen, benötigen meist keine maschinelle Beatmung. Dennoch ist es häufig - vor allem im Bereich der Intensivpflege - erforderlich, ihre Atmung zu unterstützen. Dies geschieht beispielsweise durch Erhöhung der Sauerstoffkonzentration gegenüber dem normalen Sauerstoffgehalt der Luft und durch eine geringfügige Erhöhung des Drucks (um 3 bis 15 mbar) im Vergleich zum normalen Luftdruck.

Um die Gewöhnung des Patienten an die normale Atmung zu fördern, ist es zweckmäßig, ihm kontinuierlich Atemgas mit einem weitgehend konstanten Druck zur Verfügung zu stellen. Geräte, die die Atmung spontan atmender Patienten in diesem Sinne unterstützen, werden als "CPAP" (continuous positive airway pressure)-Geräte bezeichnet.

Moderne Beatmungsgeräte haben häufig die Möglichkeit, eine CPAP-Funktion einzustellen. Bei diesen Beatmungsgeräten muß jedoch in der Regel ein einstellbares Triggerniveau zur Auslösung der Inspirationsphase überschritten werden, was den Patienten belasten kann. Außerdem sind Beatmungsgeräte technisch sehr aufwendig konstruiert und demzufolge kostspielig. Ihr Einsatz für die CPAP-Funktion ist somit unwirtschaftlich.

Ein Beatmungsgerät mit zusätzlicher CPAP-Funktion ist der GB-A-2 054 387 beschrieben. Es weist eine Atemgaskammer auf, in welche Atemgas aus einer üblichen Atemgasquelle über ein elektrisch betätigtes Ventil eingeleitet wird. Von der Atemgaskammer gelangt das Gas über einen Durchflußsensor zu dem Patienten bzw. zu einem mit einem pneumatischen Ventil, welches nur in der Expirationsphase im Betriebszustand ist, verschlossenen Auslaß. Die Steuerung der verschiedenen mit diesem Gerät möglichen Beatmungsfunktionen erfolgt über die Druckluftzufuhr in der Antriebsgaskammer. Diese wird über ein Kontrollventil von einer zentralen Kontrolleinheit gesteuert. An die Kontrolleinheit sind auch das Ventil vor der Atemgaskammer, der Durchflußsensor hinter der Atemgaskammer, ein Atemgasdruckgeber sowie ein Druckvergleicher zur Kontrolle des Differenzdruckkes zwischen Atemgaskammer und Antriebsgaskammer und ein Ventil zur Unterbrechung der Vergleichsdruckleitung durch die das Auslaßventil gesteuert wird, angeschlossen.

Die Atemgaskammer bildet dabei ein flexibles Atemgasreservoir, welches in Wirkverbindung zu dem regulierbaren Antriebsgasreservoir steht, das die Steuerungsaufgaben zur Beatmung des Patienten erfüllt. Zusätzlich zu den Komponenten des Atemgases ist ein Treibgas bzw. ein Kompressor als Energiequelle erforderlich, um den für das gewünschte Beatmungsprofil erforderlichen Druck zu erzeugen. Die Steuerung ist im wesentlichen pneumatisch, wobei das Kontrollventil über eine Venturidüse den Druck der Antriebsgaskammer steuert, diese in Wirkverbindung zu der Atemgaskammer steht und das Expirationsventil pneumatisch über den Druck in der Atemgaskammer gesteuert wird. Solche pneumatische Serienschaltungen führen erfahrungsgemäß zur erheblichen Regelverzögerungen und somit zu Druckabweichungen im Atemgasstrom.

Die in der GB-A-2 054 387 beschriebene CPAP-Funktion sieht lediglich vor, daß die zentrale Kontrolleinheit ein Sollsignal entsprechend dem gewünschten CPAP abgibt und die Druckluftzufuhr zu der Antriebsgaskammer so gesteuert wird, daß der Istwert des Atemgasdruckgebers diesem Sollwert in guter Näherung entspricht. Gegegenenfalls kann als zusätzlicher Istwert der Meßwert des Durchflußsensors hinter der Atemgaskammer zu dem Meßwert des Atemgasdruckgebers hinzu addiert werden.

Aus diesen Gründen wurden spezielle Geräte der eingangs bezeichneten Art entwickelt. Das Atemgas wird aus den in Kliniken üblicherweise vorhandenen zentralen Netzen für Luft und Sauerstoff entnommen und mittels mechanischer Atemgasmischeinheiten (sogenannter "Oxiblender") gemischt. Als Druckbegrenzer am Auslaß des Gaskanals dient üblicherweise ein Wasserschloß, wobei die Tauchtiefe der Ausströmöffnung für die Größe des Druckes bestimmend ist. Zur Dämpfung der von der Atemaktivität des Patienten herrührenden Druckschwankungen werden großvolumige, elastische Druckspeicher verwendet.

Um die erforderliche konstante Atemgaszusammensetzung sicherzustellen, muß bei den bekannten Geräten ein hoher konstruktiver Aufwand getrieben werden. Dennoch bestehen Unsicherheiten. Die Geräte müssen deswegen ständig von qualifiziertem Personal überwacht werden, oder es müssen aufwendige zusätzliche Überwachungs- und Alarmeinrichtungen vorgesehen sein.

Der Erfindung liegt daher die Aufgabe zugrunde, ein in den erwähnten Punkten verbessertes und dennoch kostengünstig herstellbares Spontanatmungs-Unterstützungsgerät zur Verfügung zu stellen.

Zur Lösung dieser Aufgabe wird bei einem Gerät der eingangs bezeichneten Art vorgeschlagen, daß die Mischeinheit eine Luftleitung und eine Sauerstoffleitung einschließt, die an einer Mischstelle zusammengeführt sind, wobei an der Luftleitung ein Luftflußgeber zur Erzeugung eines dem Luftfluß entsprechenden elektrischen Luftflußsignals und ein elektrisch steuerbares Luftventil zur zur Steuerung des Luftflusses und an der Sauerstoffleitung ein Sauerstoffflußgeber zur Erzeugung eines dem Sauerstofffluß entsprechenden elektrischen Sauerstoffflußsignals und ein elektrisch steuerbares Sauerstoffventil zur Einstellung des Sauerstoffflusses angeordnet sind, ein Atemgasdruckgeber zur Erzeugung eines dem Atemgasdruck des Gerätes entsprechenden elektrischen Signals vorgesehen ist, der am Auslaß angeordnete Druckbegrenzer als elektrisch steuerbares Auslaßventil ausgebildet ist und der Luftflußgeber, der Sauerstoffflußgeber, der Atemgasdruckgeber, das Luftventil, das Sauerstoffventil und das Auslaßventil an eine elektronische Steuereinheit angeschlossen sind, wobei die Gebersignale Eingangsgrößen für die Steuereinheit bilden und die Steuereinheit als Ausgangsgrößen Steuersignale für die Ventile abgibt.

Die Luftleitung und die Sauerstoffleitung der Mischeinheit werden an die entsprechenden zentralen Versorgungsnetze angeschlossen. Die gewünschte Sauerstoffkonzentration und der gewünschte Beatmungsdruck werden durch mehrere zusammenwirkende Regelkreise sichergestellt.

Ein erster Regelkreis (Atemgasdruck-Regelung) dient dazu, den gewünschten vorgewählten Atemgasdruck sicherzustellen. Dabei bildet der Atemgasdruckgeber den primären Istwertgeber, dessen Ist-Signal mit dem gewünschten Druck verglichen wird. Die Regelung des Drucks kann nun entweder über die Gesamtmenge des zugeführten Frischgases (Sauerstoff und Luft) mit Hilfe des Luftventils und des Sauerstoffventils erfolgen , oder die Öffnung des Auslaßventils kann entsprechend gesteuert werden.

In einem zweiten und dritten Regelkreis (Luftregelkreis und Sauerstoffregelkreis) bilden der Luftflußgeber und der Sauerstoffflußgeber jeweils Istwertgeber für die entsprechenden Flüsse , d.h. die den Geber pro Zeiteinheit durchströmende Gasmenge. In der Steuereinheit wird das Verhältnis des Luftflußsignals zu dem Sauerstoffflußsignal ermittelt und mit einem Sollwert verglichen, der der gewünschten Sauerstoffkonzentration entspricht. Weicht das Verhältnis der Flüsse von dem gewünschten Sollwert ab, so erfolgt eine entsprechende Korrektur durch Steuersignale, die die Steuereinheit an das Luftventil oder an das Sauerstoffventil abgibt. Der zweite und dritte Regelkreis bilden also eine Sauerstoff-Konzentrationsregelung.

Die Atemgas-Druckregelung bewirkt ein im wesentlichen synchrones Öffnen und Schließen des Luftventils und des Sauerstoffventils, wobei die Sauerstoffkonzentrations-Regelung das exakte Öffnungsverhalten regelt und damit zu jeder Zeit das gewünschte Mischungsverhältnis sicherstellt.

Damit in jeder Phase des Atemzyklus des Patienten ein positiver Atemgasdruck sichergestellt ist, muß der Gesamtfrischgasfluß, den man auch als "Eingangsfluß" bezeichnen kann, stets größer sein als der Maximalbedarf des Patienten.

Mit Hilfe des Luftventils und des Sauerstoffventils in Verbindung mit den entsprechenden Flußgebern ist es möglich, den Eingangsfluß dem jeweiligen Momentanbedarf des Patienten anzupassen. In der Inspirationsphase werden die Ventile geöffnet, und die Druckregelung erfolgt in erster Linie dadurch, daß man den Gesamtfrischgasfluß dem Bedarf des Patienten anpaßt.

In der Exspirationsphase können das Luftventil und das Sauerstoffventil weitgehend geschlossen werden, und die Drucksteuerung erfolgt mit Hilfe des Auslaßventils.

Diese schnelle, an den jeweiligen momentanen Atemgasbedarf des Patienten angepaßte Regelung (demand-flow-Prinzip) ist mit einem besonders geringen Gasverbrauch verbunden. Bevorzugt wird eine Regelung verwendet, bei der ein minimaler Frischgasfluß (Grundflow) nicht unterschritten wird.

Überraschenderweise ist die zunächst aufwendig erscheinende erfindungsgemäße Problemlösung insgesamt wirtschaftlich. Dies ist unter anderem darauf zurückzuführen, daß sie eine automatische Überwachung der Gerätefunktionen und Patientenparameter ermöglicht.

Beispielsweise können in der Steuereinheit Informationen gespeichert sein, in welcher Weise sich der Luftfluß in Abhängigkeit von der Stellung des Luftventils und der Sauerstofffluß in Abhängigkeit von der Stellung des Sauerstoffventils bei normaler Funktionsweise und bei störungsfreier Gasversorgung ändern müssen. Weicht das tatsächliche Regelverhalten von diesen abgespeicherten Werten um mehr als eine vorgesehene Toleranzbreite ab, wird Alarm ausgelöst.

Der Gesamtfrischgasfluß ist ein ungefähres Maß für den Atmungsbedarf des Patienten. Grobe Abweichungen können deswegen mit Hilfe des Sauerstoffflußsignals und des Luftflußsignals ermittelt werden. Auch in diesem Fall wird bei Überschreitung einer Toleranzgröße im Vergleich zu einem in der Steuereinheit abgespeicherten Sollwert Alarm ausgelöst.

Eine erheblich genauere Überwachung der Atmungsaktivität des Patienten ist bei einer bevorzugten Weiterbildung der Erfindung möglich, bei der hinter der Abzweigung der Patientenleitung ein Flußgeber zur Erzeugung eines dem ausgangsseitigen Gasfluß entsprechenden elektrischen Auslaßflußsignals vorgesehen und an die Steuereinheit angeschlossen ist. Durch Vergleich des Auslaßflußsignals mit der Summe des Luftflußsignals und des Sauerstoffflußsignals ist ein Bilanzierung möglich. Die jeweilige Differenz entspricht der Atmungsaktivität des Patienten. Somit ist bei verhältnismäßig geringem technischen Aufwand eine Bestimmung der Atmungsaktivität möglich, ohne den Atemwiderstand in der Patientenleitung zu erhöhen und dadurch den Patienten zu belasten.

Alternativ kann es jedoch auch zweckmäßig sein, gemäß einer anderen Weiterbildung der Erfindung unmittelbar in der Patientenleitung einen Patientenflußgeber vorzusehen, wobei das erzeugte Patientenflußsignal zur Überwachung der Atmungsaktivität verwendet werden kann.

Gemäß einer weiteren bevorzugten Ausführungsform ist zwischen der Mischstelle und der Abzweigung der Patientenleitung ein zusätzlicher Gesamtfrischgasflußgeber zur Erzeugung eines dem Gesamtfrischgasfluß entsprechen elektrischen Gesamtfrischgasflußsignals vorgesehen und an die Steuereinheit als zusätzliche Eingangsgröße angeschlossen. Das Gesamtfrischgasflußsignal muß bei einwandfreier Funktion des Geräts der Summe aus Luftflußsignal und Sauerstoffflußsignal entsprechen. Dadurch ist eine Überwachung der Sauerstoffkonzentration möglich, ohne eine spezielle Sauerstoffmeßsonde am Gaskanal anzuordnen. Solange die erwähnten Flußsignale übereinstimmen, ist sichergestellt, daß das Mischungsverhältnis von Luft und Sauerstoff - und damit auch die Sauerstoffkonzentration - stimmen.

Die Erfindung wird im folgenden anhand eines in der Figur schematisch dargestellten Ausführungsbeispiels näher erläutert:

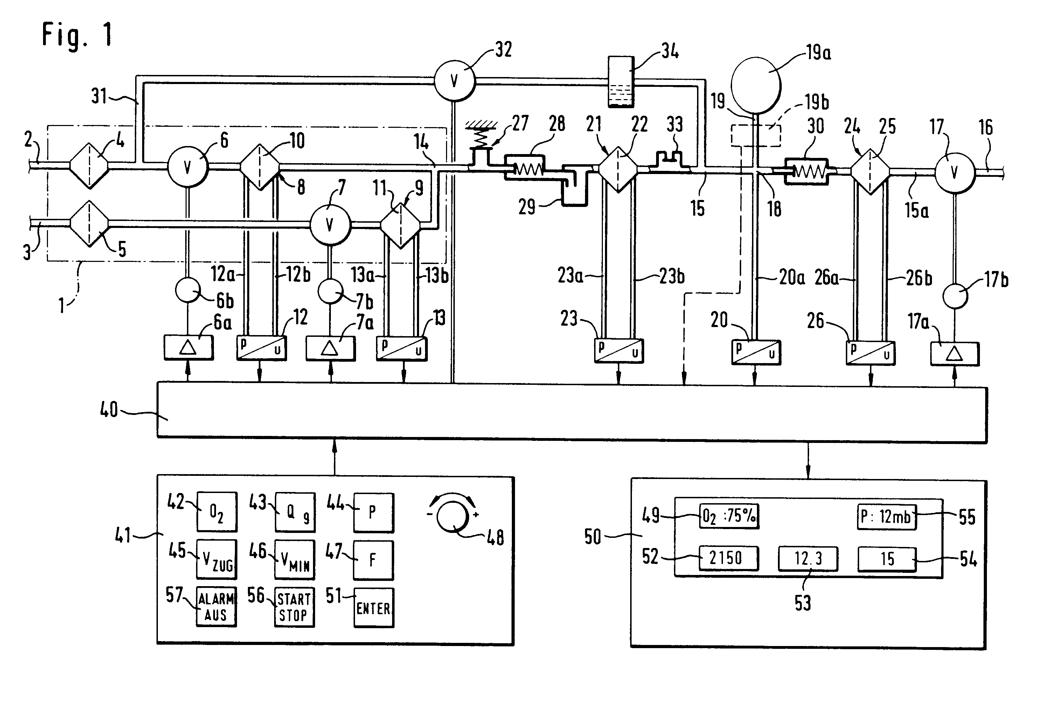

Fig. 1     zeigt ein Blockschaltbild eines erfindungsgemäßen Gerätes.

Im oberen Teil des Gerätes ist die Gasführung dargestellt. Eine Atemgasmischeinheit 1 enthält eine Luftzufuhrleitung 2 und eine Sauerstoffzufuhrleitung 3, an denen jeweils in Strömungsrichtung ein Vorfilter 4,5, ein Luftventil 6 bzw. Sauerstoffventil 7 und ein Luftflußgeber 8 bzw. Sauerstoffflußgeber 9 angeordnet sind. Der Luftflußgeber 8 und der Sauerstoffflußgeber 9 bestehen jeweils aus einem Strömungswiderstand 10 bzw. 11 und dem Differentialdruckaufnehmer 12 bzw. 13, die über Verbindungsleitungen 12a, 12b, 13a, 13b, miteinander verbunden sind. Die Bauweise solcher nach dem Differentialdruckprinzip arbeitenden Gasflußmeßeinrichtungen ist bekannt und muß hier nicht näher erläutert werden.

Die Luftleitung 2 und Sauerstoffleitung 3 werden an einer Mischstelle 14 zusammengeführt. Die Mischstelle 14 ist über einen Gaskanal 15 mit einem Auslaß 16 verbunden, der zur Atmosphäre offen ist. Vor dem Auslaß 16 ist ein Auslaßventil 17 angeordnet.

An einer Abzweigung 18 zweigt eine Patientenleitung 19 ab zu dem Patienten, dessen Lungen durch das Oval 19a symbolisiert ist. Ebenfalls von der Abzweigung 18 zweigt eine Leitung 20a zu einem Atemgasdruckgeber 20 ab. Der Atemgasdruckgeber kann auch an einer anderen Stelle angeordnet sein, die über Leitungen mit geringem Strömungswiderstand mit dem Patienten verbunden ist, so daß an der Meßstelle praktisch der gleiche Druck wie in der Patientenleitung herrscht und somit der gemessne Druck mit dem Atemgasdruck des Patienten übereinstimmt.

In dem Abschnitt des Gaskanals 15 zwischen der Mischstelle 14 und der Abzweigung 18 ist ein Gesamtfrischgasflußgeber 21 angeordnet, der aus einem Strömungswiderstand 22, einem Differentialdruckaufnehmer 23 und Verbindungsleitungen 23a, 23b besteht.

Ein Auslaßflußgeber 24, bestehend aus einem Strömungswiderstand 25, einem Differentialdruckaufnehmer 26 und Verbindungsleitungen 26a, 26b ist in dem zwischen der Abzweigung 18 und dem Auslaß 16 liegenden Abschnitt des Gaskanals 15 angeordnet.

In der Patientenleitung 19 kann, soweit eine unmittelbare Messung des Patientenflusses notwendig ist, der in der Figur gestrichelt dargestellte Patientenflußgeber 19b vorgesehen sein.

Weiter befinden sich an dem Gaskanal ein Sicherheits-Überdruckventil 27, ein erstes Rückschlagventil 28, ein Befeuchter 29 und ein zweites Rückschlagventil 30. Die Rückschlagventile dienen in bekannter Weise dazu, das Wiedereinatmen verbrauchter Atemluft (Rückatmung) zu verhindern.

Zur Betätigung der Ventile 6,7,17 dient jeweils ein Stellglied 6a,7a und 17a, das einen elektromagnetischen Antrieb 6b,7b und 17b steuert, der das eigentliche Ventil betätigt.

In der Luftleitung 2 mündet vor dem Luftventil 6 eine Zusatzleitung 31, durch die Luft, gesteuert durch ein Ventil 32 entnommen werden kann. Die Luft dient als Treibgas, beispielsweise zur Erzeugung eines Aerosols eines von dem Patienten benötigten Medikaments. Ein zu diesem Zweck vorgesehener Vernebler ist mit 34 bezeichnet.

Die Gasführung in dem Gerät wird mit Hilfe einer zentralen elektronischen Steuereinheit 40 gesteuert. An diese sind als Eingangsgrößen der Luftflußgeber 8, der Sauerstoffflußgeber 9, der Gesamtfrischgasflußgeber 22, der Auslaßflußgeber 24, der Atemgasdruckgeber 20 und ggf. der Patientenflußgeber 19b angeschlossen.

Weitere Eingangsgrößen erhält die Zentraleinheit 40 von einer Eingabeeinheit 41, die Sollwerte für die einzustellenden Parameter und Alarmgrenzwerte liefert. Im dargestellten Fall ist für jeden derartigen Wert eine Taste vorgesehen, nämlich:

Taste 42      für den Sollwert der Sauerstoffkonzentration

Taste 43      für den Sollwert des minimalen Frischgasflusses (Grundflow)

Taste 44      für den Sollwert des Atemgasdruckkes

Taste 45      für die Alarmgrenzwerte des Atemzugvolumens

Taste 46      für die Alarmgrenzwerte des Atemminuten-Volumens

Taste 47      für die Alarmgrenzwerte der Atemfrequenz pro Minute

Die Einstellung dieser Werte erfolgt dabei jeweils in der Weise, daß zunächst die Taste gedrückt und dann mit Hilfe eines Einstellknopfes 48 der Wert eingestellt wird, der auf einem entsprechenden Display 49,52,53,54,55 einer Ausgabeeinheit 50 angezeigt wird. Wenn der gewünschte Wert eingestellt ist, wird eine Enter-Taste 51 betätigt, um den Wert in die Steuereinheit 40 zu überführen.

Weitere Tasten der Eingabeeinheit 41 dienen zum Ein- und Ausschalten des Gerätes (Start-Stop-Taste 56) und zum Löschen eines Alarmsignals (Alarm-Aus-Taste 57).

An die Steuereinheit sind weiterhin die Stellglieder 6a,7a und 17a der Ventile 6,7,17 angeschlossen, wobei die Steuereinheit als Ausgangsgrößen Steuersignale an die Stellglieder abgibt.

Die Ausgangsgrößen der Steuereinheit 40 liegen an der Ausgabeeinheit 50 an, wo sie mit Hilfe entsprechender digitaler Anzeigen angezeigt werden; dargestellt sind Anzeigefelder für die Sauerstoffkonzentratton 49, für das Atemzugvolumen 52, für das Atemminutenvolumen 53, für die Atemfrequenz 54 und für den Atemgasdruck 55.

Im praktischen Betrieb werden zunächst mit Hilfe der Tasten 42,43 und 44 und dem Einstellknopf 48 die entsprechenden Sollwerte und gewünschtenfalls mit Hilfe der Tasten 45,46 und 47 die entsprechenden Alarmgrenzen eingestellt. Vor dem Einschalten müssen die Luftleitung 2 und die Sauerstoffleitung 3 an die entsprechenden zentralen Netze der Klinik angeschlossen sein. Der Patient wird in üblicher Weise über einen geeigneten Adapter mit der Patientenleitung 19 verbunden.

Nach Einschalten mit Hilfe der Taste 56 werden die Ventile 6 und 7 geöffnet und die beiden Gase über die Vorfilter 4,5 und die Strömungswiderstände 8,9 der Mischstelle 14 zugeleitet. An den Strömungswiderständen werden die stromaufwärts bzw. stromabwärts anstehenden Drucke jeweils über die Leitungen 12a,12b und 13a,13b den Differentialdruckaufnehmern 12,13 zugeführt, die dem Istwert des jeweiligen Gasflusses entsprechende elektrische Signale erzeugen und an die Steuereinheit 40 übermitteln. In der Steuereinheit 40 werden diese Fluß-Istwerte mit den Fluß-Sollwerten verglichen, die zuvor anhand der in der Eingabeeinheit 41 eingegebenen Werte für den Gesamtfluß und die Sauerstoffkonzentration berechnet wurden. Werden Abweichungen zwischen Soll- und Istwerten festgestellt, so werden das Luftventil 6 und das Sauerstoffventil 7 so angesteuert, daß die Istwerte den Sollwerten angeglichen werden.

Wie weiter oben dargelegt, erfüllen der Luftregelkreis (Luftventil 6, Luftflußgeber 8) und der Sauerstoffregelkreis (Sauerstoffventil 7, Sauerstoffflußgeber 9) dabei eine doppelte Funktion. Zum einen wird die Atemgaszusammensetzung durch das Verhältnis des Sauerstoffflusses zu dem Luftfluß bestimmt und entsprechend dem eingestellten Sollwert geregelt. Zum anderen dienen beide Regelkreise aber auch zur Einstellung des konstanten Atemgasdruckes, wie im folgenden näher erläutert wird.

Nach der Aufbereitung des Atemgasgemisches in dem Atemgasmischer 1 gelangt das Atemgas zu der Abzweigung 18 , an der die Patientenleitung 19 abzweigt und Atemgasdruckgeber 20 angeschlossen ist. Weiter strömt das Atemgas über das Rückschlagventil 30, den Strömungswiderstand 25 des Auslaßflußgebers 24 und das Auslaßventil 17 zu dem Auslaß 16.

Dem in dem Gaskanal 15 strömenden Fluß des Atemgases wird der Atemfluß des Patienten (Patientenleitung 19) hinzuaddiert, so daß in dem in Strömungsrichtung hinter der Abzweigung 18 liegenden Expirationszweig 15a des Gaskanals 15 ein im Atmungsrhythmus schwankender, aber stets zum Auslaß gerichteter Fluß entsteht. Das Auslaßventil 17 bildet je nach seiner Stellung einen veränderlichen Strömungswiderstand und beeinflußt damit den Druck an der Abzweigung 18 und damit den Atemgasdruck. Dieser Druckistwert wird von dem Atemgasdruckgeber 20 gemessen und steht der Steuereinheit 40 als elektrische Spannung zur Verfügung. In der Steuereinheit wird der Druckistwert mit dem Drucksollwert (Eingabeeinheit 41, Taste 44) verglichen. Das Auslaßventil 17 wird aufgrund der entsprechenden Ausgangsgröße der Steuereinheit 40 über das Stellglied 17a so gesteuert, daß der Druckistwert dem Drucksollwert nachgeführt wird.

Das Auslaßventil 17 dient bei der hier beschriebenen bevorzugten Funktionsweise zur kurzfristigen Regelung des Atemgasdruckes. Es ist deshalb vorzugsweise ein sowohl in Öffnungsrichtung als auch in Schließrichtung elektrisch betätigtes Ventil, das eine kleine Zeitkonstante von vorzugsweise weniger als 0,1 sec, bevorzugt weniger als 0,02 sec sowohl bezüglich des Schließverhaltens als auch bezüglich des Öffnungsverhaltens aufweist. Die Zeitkonstante ist dabei, wie üblich, als die Zeit definiert, die benötigt wird, um das Ventil 63 % seines gesamten Stellweges zu bewegen.

Zur Aufrechterhaltung des CPAP-Druckes ist es erforderlich, daß im Exspirationszweig 15a stets ein in Richtung Ausgang 16 gerichteter Fluß herrscht. In der Exspirationsphase wird dieser Fluß durch den Gasstrom aus der Patientenleitung 19 sichergestellt. Der in der Leitung 15 vor der Abzweigung 18 strömende Gesamtfrischgasfluß kann in dieser Phase mit Hilfe der Atemgasmischeinheit 1 nahezu auf 0 reduziert werden.

Bevorzugt werden als Luftventil 6 und als Gasventil 7 aus Sicherheitsgründen elektromagnetisch öffnende, durch Federkraft schließende Ventile eingesetzt. Die Zeitkonstante ihres Öffnungsverhaltens und ihres Schließverhaltens sollte ebenso wie bei dem Auslaßventil 17 kürzer sein als die Periodendauer der menschlichen Atmung. Vorzugsweise ist sie kleiner als 0,1 sec., besonders bevorzugt kleiner als 0,02 sec. Durch diese kleinen Zeitkonstanten besteht die Möglichkeit, den die Atemgasmischeinheit 1 verlassenden Gesamtfrischgasfluß ständig dem aktuellen Bedarf der spontanen Atmung des Patienten anzugleichen.

Die Steuereinheit 40 arbeitet bevorzugt mit Hilfe eines Microprozessors auf Basis eines entsprechenden Programms. Aufgrund der hier gegebenen Informationen ist es dem Fachmann ohne weiteres möglich, eine Steuereinheit, die die beschriebenen Funktionen erfüllt, zu entwerfen. Dies kann gegebenenfalls auch mit Hilfe einer entsprechenden speziellen elektronischen Schaltung ohne Verwendung eines Microprozessors erfolgen, jedoch ist die Microprozessor-Steuerung bevorzugt.

**Patentansprüche**

1. Gerät zur Unterstützung der spontanen Atmung eines Patienten mit

einer Atemgasmischeinheit (1) zur Mischung von Luft und Sauerstoff in einem gewünschten Verhältnis zur Versorgung des Patienten mit Atemgas einer einstellbaren Sauerstoffkonzentration,

einem Auslaß (16), an dem ein Druckbegrenzer angeordnet ist,

einem Gaskanal (15), der die Atemgasmischeinheit (1) mit dem Auslaß (16) verbindet, und

einer Patientenleitung (19), die an einer zwischen der Atemgasmischeinheit (1) und dem Auslaß (16) angeordneten Abzweigung (18) von dem Gaskanal (15) abzweigt,

**dadurch gekennzeichnet, daß**

die Mischeinheit (1) eine Luftleitung (2) und eine Sauerstoffleitung (3) einschließt, die an einer Mischstelle (14) zusammengeführt sind, wobei an der Luftleitung ein Luftflußgeber (8) zur Erzeugung eines dem Luftfluß entsprechenden elektrischen Luftflußsignals und ein elektrisch steuerbares Luftventil (6) zur Einstellung des Luftflusses und an der Sauerstoffleitung (3) ein Sauerstoffflußgeber (9) zur Erzeugung eines dem Sauerstofffluß entsprechenden elektrischen Sauerstoffflußsignals und ein Sauerstoffventil (7) zur Einstellung des Sauerstoffflusses angeordnet sind,

ein Atemgasdruckgeber (20) zur Erzeugung eines dem Atemgasdruck des Gerätes entsprechenden elektrischen Signals vorgesehen ist,

der am Auslaß (16) angeordnete Druckbegrenzer als elektrisch steuerbares Auslaßventil (17) ausgebildet ist , und

der Luftflußgeber (8), der Sauerstoffflußgeber (9) , der Atemgasdruckgeber (20), das Luftventil (6), das Sauerstoffventil (7) und das Auslaßventil (17) an eine elektronische Steuereinheit (40) angeschlossen sind, wobei die Gebersignale Eingangsgrößen für die Steuereinheit (40) bilden und die Steuereinheit (40) als Ausgangsgrößen Steuersignale für die Ventile abgibt.

2. Gerät nach Anspruch 1, **dadurch gekennzeichnet,** daß an dem Gaskanal (15) hinter der Abzweigung (18) der Patientenleitung (19) ein Auslaßflußgeber (24) zur Erzeugung eines dem ausgangsseitigen Gasfluß entsprechenden elektrischen Auslaßflußsignals vorgesehen und an die Steuereinheit (40) angeschlossen ist.

3. Gerät nach Anspruch 1, **dadurch gekennzeichnet,** daß an der Patientenleitung (19) ein Patientenflußgeber (19b) zur Erzeugung eines dem Patientenfluß entsprechenden elektrischen Patientenflußsignals vorgesehen und an die Steuereinheit (40) angeschlossen ist.

4. Gerät nach Anspruch 1, **dadurch gekennzeichnet,** daß zwischen der Mischstelle (14) und der Abzweigung (18) der Patientenleitung (19) ein zusätzlicher Gesamtfrischgasflußgeber (22) zur Erzeugung eines dem Gesamtfrischgasfluß entsprechenden elektrischen Gesamtfrischgasflußsignals vorgesehen und an die Steuereinheit (40) als Eingangsgröße angeschlossen ist.

5. Gerät nach Anspruch 1, **dadurch gekennzeichnet,** daß das Luftventil (6) und das Sauerstoffventil (7) elektromagnetisch öffnende, durch Federkraft schließende Ventile sind.

6. Gerät nach Anspruch 1, **dadurch gekennzeichnet,** daß das Luftventil (6) und das Sauerstoffventil (7) sowohl bezüglich des Schließverhaltens als auch bezüglich des Öffnungsverhaltens eine Zeitkonstante kleiner als 0,1 sec., bevorzugt kleiner als 0,02 sec. haben.

7. Gerät nach Anspruch 1, **dadurch gekennzeichnet,** daß das Auslaßventil (17) ein sowohl in Öffnungsrichtung als auch in Schließrichtung elektrisch betätigtes Ventil ist.

8. Gerät nach Anspruch 1, **dadurch gekennzeichnet,** daß das Auslaßventil (17) sowohl bezüglich des Schließverhaltens als auch bezüglich des Öffnungsverhaltens eine Zeitkonstante kleiner als 0,1 sec., bevorzugt kleiner als 0,02 sec. hat.

## Claims

1. Apparatus for assisting the spontaneous breathing of a patient with respiratory gas mixing unit (1) for the mixing of air and oxygen in a desired ratio for the provision of the patient with respiratory gas of an adjustable oxygen concentration, an outlet (16) on which is arranged a pressure control, a gas duct (15) which connects the respiratory gas mixing unit (1) with the outlet (16) and a patient line (19) which branches off from the gas duct (15) on a junction (18) arranged between the respiratory gas mixing unit (1) and the outlet (16), characterised in that the mixing unit (1) includes an air conduit (2) and an oxygen conduit (3) which are combined at a mixing area (14), whereby, on the air conduit, are provided an air flow sensor (8) for the generation of an electrical air flow signal corresponding to the air flow and an electrically-controllable air valve (6) for the adjustment of the air flow and, on the oxygen conduit (3), an oxygen flow sensor (9) for the generation of an electrical oxygen flow signal corresponding to the oxygen flow and an oxygen valve (7) for the adjustment of the oxygen flow, a respiratory gas pressure sensor (20) for the generation of an electrical signal corresponding to the respiratory gas pressure of the apparatus, the pressure control Provided on the outlet (16) is constructed as an electrically controllable outlet valve (17) and the air flow sensor (8), the oxygen flow sensor (9), the respiratory gas sensor (20), the air valve (6), the oxygen valve (7) and the outlet valve (17) are connected to an electronic control unit (40), whereby the sensor signals form input values for the control unit (40) and the control unit (40) emits, as output values, control signals for the valves.

2. Apparatus according to claim 1, characterised in that, on the gas duct (15) behind the junction (18) of the patient line (19), is provided an outlet flow sensor (24) for the generation of an electrical outlet flow signal corresponding to the outlet side gas flow and is connected to the control unit (40).

3. Apparatus according to claim 1, characterised in that, on the patient line (19), is provided a patient flow sensor (19b) for the generation of an electrical patient flow signal corresponding to the patient flow and is connected to the control unit (40).

4. Apparatus according to claim 1, characterised in that, between the mixing area (14) and the jucntion (18) of the patient line (19), there is provided an additional total fresh gas flow sensor (22) for the generation of an electrical total fresh gas flow signal corresponding to the total fresh gas flow and is connected to the control unit (40) as input value.

5. Apparatus according to claim 1, characterised in that the air valve (6) and the oxygen valve (7) are electromagnetically opening valves closed by spring force.

6. Apparatus according to claim 1, characterised in that the air valve (6) and the oxygen valve (7) have a time constant smaller than 0.1 sec., preferably smaller than 0.02 sec., not only with regard to the closing behaviour but also with regard to the opening behaviour.

7. Apparatus according to claim 1, characterised in that the outlet valve (17) is an electrically actuated valve not only in the opening direction but also in the closing direction.

8. Apparatus according to claim 1, characterised in that the outlet valve (17) has a time constant smaller than 0.1 sec., preferably smaller than 0.02 sec., not only with regard to the closing behaviour but also with regard to the opening behaviour.

## Revendications

1. Appareil d'assistance à la respiration spontanée d'un patient, muni d'une unité mélangeuse de gaz respiratoire (1) destinée à mélanger de L'air et de l'oxygène dans une proportion recherchée, pour alimenter le patient en gaz respiratoire présentant une concentration en oxygène qui est réglable, d'une évacuation (16) sur laquelle est disposé un limiteur de pression, d'un canal de gaz (15) qui relie l'unité mélangeuse de gaz respiratoire (1) à l'évacuation (16), et d'une canalisation reliée au patient (19), qui bifurque du canal de gaz (15) en une dérivation disposée entre l'unité mélangeuse de gaz respiratoire (1) et l'évacuation (16), caractérisé en ce que l'unité mélangeuse (1) comprend une canalisation d'air (2) et une canalisation d'oxygène (3) qui aboutissent à une station de mélange (14), un capteur d'écoulement d'air (8) destiné à la production d'un signal électrique d'écoulement d'air correspondant à l'écoulement de l'air ainsi qu'une soupape d'air (6), commandable électriquement et destinée au réglage de l'écoulement de l'air, étant disposés sur la canalisation d'air, un capteur d'écoulement d'oxygène (9) destiné à la production d'un signal électrique correspondant à l'écoulement de l'oxygène et une soupape d'oxygène (7) destinée au réglage de l'écoulement de l'oxygène étant disposés sur la canalisation d'oxygène (3), en ce qu'un capteur de pression du gaz respiratoire (20), destiné à la production d'un signal électrique correspondant à la pression du gaz respiratoire de l'appareil, est prévu, en ce que le limiteur de pression disposé sur l'évacuation (16) est conçu en tant que soupape d'échappement (17) réglable électriquement, et en ce que le capteur d'écoulement d'air (8), le capteur d'écoulement d'oxygène (9), le capteur de pression du gaz respiratoire (20), la soupape d'air (6), la soupape d'oxygène (7) et la soupape d'échappement (17) sont raccordés à un organe de commande électronique (40), les signaux des capteurs formant des grandeurs d'entrée pour l'organe de commande (40) et l'organe de commande (40) donnant des signaux de commande pour les soupapes, en tant que grandeurs de sortie.

2. Appareil selon la revendication 1, caractérisé en ce qu'un capteur d'écoulement d'échappement (24), destiné à la production d'un signal électrique de l'écoulement d'échappement correspondant à l'écoulement du gaz situé du côté de la sortie, est prévu sur le canal de gaz (15), derrière la dérivation (18) de la canalisation reliée au patient, et raccordé à l'organe de commande (40).

3. Appareil selon la revendication 1, caractérisé en ce qu'un capteur d'écoulement vers le patient (19b), destiné à la production d'un signal électrique de l'écoulement vers le patient correspondant à l'écoulement vers le patient, est prévu sur la canalisation reliée au patient (19) et raccordé à l'organe de commande (40).

4. Appareil selon la revendication 1, caractérisé en ce qu'entre la station de mélange (14) et la dérivation (18) de la canalisation reliée au patient (19) est prévu un capteur supplémentaire d'écoulement de l'ensemble du gaz frais (22), destiné à la production d'un signal électrique de l'écoulement de l'ensemble du gaz frais correspondant à l'écoulement de l'ensemble du gaz frais et en ce qu'il est raccordé à l'organe de commande (40) en tant que grandeur d'entrée.

5. Appareil selon la revendication 1, caractérisé en ce que la soupape d'air (6) et la soupape d'oxygène (7) sont des soupapes qui s'ouvrent de façon électromagnétique et se ferment par effet de ressort.

6. Appareil selon la revendication 1, caractérisé en ce que la soupape d'air (6) et la soupape d'oxygène (7) possèdent une constante de temps inférieure à 0,1 s., de préférence inférieure à 0,02 s., aussi bien par rapport à leur comportement pendant la fermeture qu'à leur comportement pendant l'ouverture.

7. Appareil selon la revendication 1, caractérisé en ce que la soupape d'échappement (17) est une soupape actionnée électriquement aussi bien dans le sens de l'ouverture que dans celui de la fermeture.

8. Appareil selon la revendication 1, caractérisé en ce que la soupape d'échappement (17) possède une constante de temps inférieure à 0,1 s., de préférence inférieure à 0,02 s., aussi bien par rapport à son comportement pendant la fermeture qu'à son comportement pendant l'ouverture.

Fig. 1